# EUROPÄISCHE PATENTSCHRIFT

(11) **EP 0 509 338 B1**
(45) Veröffentlichungstag und Bekanntmachung des Hinweises auf die Patenterteilung: **16.08.1995**
(21) Anmeldenummer: 92105737.8
(22) Anmeldetag: 03.04.1992
(51) Int. Cl.: A61K 9/127, A61K 7/00

(54) **Herstellung und Verwendung stabiler kleinpartikulärer Liposomenzubereitungen**
Manufacture and use of stable preparations of small-sized liposomes
Fabrication et utilisation de préparations stables de liposomes de petite taille

(30) Priorität: 12.04.1991 DE 4111982
(43) Veröffentlichungstag der Anmeldung: 21.10.1992
(73) Patentinhaber: Merz & Co. GmbH & Co., D-60318 Frankfurt (DE)
(72) Erfinder: Mentrup, Edgar, Dr., W-6500 Mainz (DE); Michel, Cristoph, W-6050 Offenbach (DE); Purmann, Thomas, Dr., W-8750 Aschaffenburg (DE)
(74) Vertreter: Beil, Hans Chr., Dr.

(56) Entgegenhaltungen:
- WO-A-87/06460
- WO-A-90/12565

## Beschreibung

Die vorliegende Erfindung betrifft das in den Ansprüchen gekennzeichnete Verfahren zur Herstellung stabiler Liposomendispersionen und deren Verwendung.

Liposomen sind, wie allgemein bekannt, kleine Vesikel mit Bilayerstruktur. Sie finden im pharmazeutischen Bereich Anwendung als Arzneistoffträger, z.B. zur parenteralen oder topischen Applikation.

Wirkstoffe mit unterschiedlichen chemisch-physikalischen Eigenschaften können in Liposomen eingearbeitet werden. Wasserlösliche Wirkstoffe sind im Innenraum der Vesikel und zwischen den Bilayerlamellen zu finden. Lipophilere Substanzen dagegen interkalieren in den Bilayer. Arbeitet man daher größere Mengen solcher Wirkstoffe in Liposomen ein, kann sich der Durchmesser der Vesikel deutlich vergrößern. In diesem Fall können sich pharmakokinetische und somit auch therapeutische Eigenschaften wesentlich ändern. Kleine Liposomen sind häufig vorteilhaft, da sie nicht durch das reticulo-endotheliale System (RES) erkannt werden und somit nicht in Leber oder Niere aus dem Blutkreislauf ausgefiltert werden. Dadurch können Plasmaspiegel über längere Zeit erhalten werden. Beispielsweise wurde für solche Liposomen eine Halbwertszeit von über 20 Stunden ermittelt (vgl. J. Senior et al., Biochim. Biophys. Acta 839, 1 (1985)). Dies bedeutet eine erhöhte Verfügbarkeit eines verkapselten Wirkstoffs am potentiellen Wirkungsort, z.B. einem Tumorgewebe. Denn das zirkulierende Liposomendepot wird durch enzymatischen Abbau und Wechselwirkung mit anderen Blutbestandteilen nur langsam abgebaut. Dabei wird der verkapselte Wirkstoff freigegeben und kann für therapeutische Zwecke zur Verfügung stehen.

Große Vesikel dagegen werden durch das RES, besonders durch die Kupferzellen der Leber, als Fremdkörper erkannt und zerstört. Bereits wenige Minuten nach der i.v.-Applikation solcher Partikel sind zum Teil über 90% der Partikel ausgefiltert. So wurde nach der Applikation von 400 nm großen Liposomen eine Halbwertszeit von ca. 10 min gefunden, bei Partikeln der Größe 1000 nm betrug sie nur noch ca. 5 min (vgl. J.Senior et al, a.a.O., und P.O.Lelkes, Liposome Technology, Vol. 1). Auch nach topischer Applikation können kleine wirkstoffhaltige Vesikel u.U. durch eine verbesserte Penetration z.B. längs der Haarfollikel eine verbesserte Wirkung zeigen. Ferner besteht die Möglichkeit einer Interaktion mit den in den interzellulären Zwischenräumen befindlichen natürlichen Bilayerstrukturen (vgl. W. Curatolo, Pharm. Res. 4, 271-341 (1987), G. Grubauer et al., J. Lipid Res. 30, 89-96 (1989)). Zudem kann bei einer unilamellaren Struktur der Vesikel im Vergleich zu multilamellaren großen Vesikeln statistisch gesehen eine vermehrte Interaktion mit anderen Zellen erfolgen. Dadurch ist ein erleichterter Transfer von Membranbestandteilen, z.B. verkapselten Wirkstoffen, möglich.

In der FR-A 2 591 105 werden pharmazeutische Zusammensetzungen auf der Basis von lipidhaltigen multilamellaren Vesikeln zur dermatologischen oder kosmetischen Anwendung beschrieben, welche ein Retinoid oder ein Analogon hiervon enthalten. Durch diese Kombination sollen Irritationen auf der Haut vermieden werden. Als Vesikelbestandteile kommen dabei hydrierte Lipide und z.B. Cholesterol oder Sitosterol, zum Einsatz. Die daraus hergestellten Liposome weisen jedoch eine hohe Teilchengröße und die damit verbundenen Nachteile auf. Insgesamt sind daher Partikel geringer Größe erstrebenswert.

Neben der Teilchengröße ist auch die Stabilität der Liposomenzubereitung von entscheidender Bedeutung für eine therapeutische oder kosmetische Verwendung derselben.

Aus galenischer Sicht problematisch und für therapeutische Anwendungen häufig limitierend ist dabei die Instabilität von Liposomenzubereitungen, und zwar einerseits hinsichtlich der Partikel selbst und andererseits hinsichtlich des eingearbeiteten Wirkstoffs. Im Vordergrund steht zunächst eine Zersetzung des Wirkstoffs. Art und Geschwindigkeit der Zersetzung sind für jeden Wirkstoff spezifisch. Oxidative Prozesse können durch geeignete Antioxidantien, wie Butylhydroxytoluol oder Vitamin E, reduziert werden. pH-abhängige Hydrolyseprozesse sind durch eine geeignete Pufferung reduzierbar. Bei hydrolyseempfindlichen Wirkstoffen kann durch den verstärkten Kontakt der Substanz mit Wasser eine erhöhte Hydrolyse auftreten, jedoch besteht durch ausreichende Einlagerung in den Bilayer die Möglichkeit der Stabilisierung. Entscheidend für die Wirkstoffstabilität sind die jeweilige Zersetzungsreaktion und die physiko-chemischen Eigenschaften der Substanz.

Neben der Wirkstoffzersetzung kann, wie oben erwähnt, eine Beeinträchtigung der Liposomen selbst durch Lipidoxidation, Lipidhydrolyse und Teilchenaggregation auftreten. Die Lipidoxidation kann durch den Zusatz von Antioxidantien vermindert werden, wie beispielsweise von C.A. Hunt, S. Tsang., in Int.J.Pharm. 8, 101 (1981) oder von A.W.T. Konings in Liposome Technology, Vol. I, beschrieben. Alternativ besteht die Möglichkeit, für Liposomen Lezithine mit gesättigten Fettsäureestern einzusetzen, die nicht oxidiert werden können (vgl. P. Kibat, Dissertation, Universität Heidelberg 1987). Eine Hydrolyse der Lezithine zu Lysolezithinen kann zusätzlich durch Pufferung der Systeme im Bereich von pH 6-7 verringert werden (vgl. S. Froeckjear et al., Optimization of Drug Delivery, A. Benzon Symposium 17, Kopenhagen (1982)).

Darüberhinaus kann vor allem bei kleinen unilamellaren Vesikeln bereits nach wenigen Wochen eine unerwünschte Teilchenaggregation auftreten. Dadurch verändern sich die Eigenschaften der Vesikel während der Laufzeit eines Produktes. Bisher bekannte Möglichkeiten gegen solche Veränderungen sind wiederum der Einsatz hydrierter (gesättigter) Lipide oder von Cholesterol bis zu 50 Mol-% bezüglich der Lipidgesamtmenge (vgl. P. Kibat, a.a.O.). Nachteilig erwies sich dabei jedoch die Zunahme der Teilchengröße bei Verwendung hydrierter Lipide. Zudem weisen hydrierte Lipide meist eine erhöhte Phasenübergangstemperatur von 50 bis 60°C auf, während diese bei herkömmlichen Lezithinen, z.B. bei Phosphatidylcholinen, -15 bis 30°C beträgt (vgl. B.D.Ladbrooke, D. Chapman, Chem. Phys. Lipids 3, 304 (1969)). Zur Herstellung von Liposomen muß aufgrund des erforderlichen fluiden Membranzustandes oberhalb der Phasenübergangstemperatur gearbeitet werden. Dabei ist die Handhabung der Materialien im Falle hydrierter Lipide technisch erschwert. Ferner sind hydrierte Lipide, da es sich um synthetische Substanzen handelt, aus toxikologischer Sicht bedenklicher als native Lezithine aus Eiern oder Sojapflanzen.

Bei einem Einsatz von Cholesterol zur Stabilisierung ist der meist reduzierte Einbau lipophiler Wirkstoffe aufgrund einer kompetitiven Verdrängung durch Cholesterol problematisch (vgl. E. Mentrup, Dissertation, Universität Heidelberg, 1988). In Abhängigkeit von den Wirkstoffeigenschaften ist u.U. kein Einbau von therapeutisch wirksamen Mengen möglich.

Die WO 87/06460 offenbart Liposomendispersionen, hergestellt durch Verdampfung oder Anwendung von Ultrahomogenisation, wobei Lezithin und maximal 1-15% Collagenhydrolysat gemischt werden. Gemäß dieser Druckschrift soll ein Wert von 15 % nicht überschritten werden, um die Permeabilität, d.h. die Instabilitätsrate, nicht zu erhöhen.

Aus der WO 90/12565 ist ein Verfahren zur Herstellung von Liposomenformulierungen durch Rühren und Mischen von Lezithin und 1-30 % Collagenhydrolysat bekannt. Weiterhin ist die Zugabe einer starken Base zwingend erforderlich. Der Einsatz des Collagenhydrolysats erfolgt unter dem Gesichtspunkt des schnellen Quellens zwecks leichter Weiterverarbeitung.

Aufgabe vorliegender Erfindung ist es, Liposomenzubereitungen bereitzustellen, die eine kleine, im wesentlichen konstante, Teilchengröße aufweisen und insgesamt stabil sind, wobei keine Zersetzung eines eventuell eingearbeiteten Wirkstoffs erfolgt. Ferner sollen sowohl bei der topischen therapeutischen als auch bei der kosmetischen Anwendung keine toxischen Irritationen auf der Haut auftreten.

Diese Aufgabe wird erfindungsgemäß durch ein Verfahren gelöst, wobei man eine Dispersion, enthaltend 0,5 bis 35 Gew.-%, bezogen auf die Dispersion, an Lezithin und fettsäureverestertem Collagenhydrolysat, übliche Zusatzstoffe sowie Wasser, wobei die Vesikel aus einer Mischung aus einem oder mehreren Lezithinen mit fettsäureverestertem Collagenhydrolysat aufgebaut sind und eine Teilchengröße von 20 bis 150 nm aufweisen, herstellt, indem
a) das Lezithin mit mehr als 15 bis 90%, vorzugsweise 20 bis 80%, fettsäureverestertem Collagenhydrolysat (bezogen auf die Gesamtmenge an Lezithin und fettsäureverestertem Collagenhydrolysat), und gegebenenfalls mit einem pharmazeutischen Wirkstoff sowie üblichen Zusatzstoffen in einer wäßrigen Phase bei einem pH-Wert von 5 bis 7 in Gegenwart lediglich eines für diesen pH-Wert-Bereich wirksamen Puffers dispergiert wird und die erhaltene Dispersion in einem Hochdruckhomogenisator zerkleinert und homogenisiert wird oder
b) zur Dispergierung eine Mischung aus dem Lezithin, dem fettsäureveresterten Collagenhydrolysat in den angegebenen Mengen und gegebenenfalls pharmazeutischem Wirkstoff und üblichen Zusatzstoffen sowie Ethanol in eine wäßrige Phase bei pH 5-7 in Gegenwart lediglich eines für diesen pH-Wert-Bereich wirksamen Puffers injiziert wird.

Als Liposomenbaustein besonders geeignet ist hierbei Soja- oder Eilezithin. Neben diesen beiden nativen Lezithinen eignen sich besonders auch andere synthetisch hergestellte oder native C₁₆-C₂₀-Phosphatidylcholine, wobei der Fettsäureanteil gesättigt oder ungesättigt sein kann. Hierin werden vorzugsweise 20 bis 80% des fettsäureveresterten Collagenhydrolysats eingearbeitet. Insbesondere bevorzugt ist dabei ölsäureverestertes Collagenhydrolysat (ÖKH). Dieses Material ist ein amphiphiles Tensid, das in fester Form vorliegt. Es wird zwar in pharmazeutischen und kosmetischen Cremes schon als Stabilisator verwendet, wird jedoch zur Stabilisierung von Liposomen bisher nicht eingesetzt.

Die Gesamtkonzentration von Lezithin und Collagenhydrolysat in der erhaltenen wäßrigen Dispersion liegt zwischen 5 und 350 mg/ml, vorzugsweise zwischen 20 und 180 mg/ml. Dies entspricht etwa 0,5 bis 35% bzw. bevorzugt 2-18% Lezithin/Collagenhydrolysat in der Liposomenzubereitung. Durch die Verwendung von Collagenhydrolysat, insbesondere von ÖKH, werden die erzeugten Liposomen deutlich kleiner als ohne diesen Zusatz, was die oben beschriebenen Vorteile mit sich bringt. Dieser Effekt wurde bisher bei wirkstoffhaltigen Liposomen nicht erzielt. Lediglich bei Wirkstoff-Freiheit kann durch den Zusatz einiger anderer Substanzen mit amphiphilem Charakter eine Teilchengrößenverringerung erreicht werden. In der nachfolgenden Tabelle 1 ist der Einfluß verschiedener, in der Liposomentechnologie üblicher Substanzen auf die Teilchengröße der mit einem Hochdruckhomogenisator, also nach jeweils gleichem Verfahren, hergestellten Liposomen aufgeführt. Stellt man andererseits große Liposomen aus Sojalezithin durch die Schüttelmethode her (vgl. A.D. Bangham et al., J. Mol. Biol. 13, 238 (1965)), so sind die Liposomen bei dem Zusatz einer amphiphilen Substanz (z.B. bei Cholesterin) deutlich größer als die ursprünglichen Teilchen. Der erfindungsgemäße Zusatz von Collagenhydrolysat und besonders von ÖKH führt jedoch auch hier zu einer deutlichen Verringerung der Teilchengröße, wie nachstehend aufgezeigt. Der Einfluß auf die Teilchengröße ist also verfahrensunabhängig und nur substanzabhängig.

**Tabelle 1**

| Einfluß verschiedener Substanzen auf die Teilchengröße von wirkstoff-freien Lipidvesikeln (Herstellung: Hochdruckhomogenisator, 30 Min. 1,4·10⁸ Pa) | | | |
|---|---|---|---|
| Konzentration Sojalezithin (mg/ml) | Lipidzusatz | Konzentration Lipidzusatz (mg/ml) | Teilchengröße nm |
| 100 | - | - | 40±10 |
| 80 | ÖKH | 20 | 25±4 |
| 80 | mittelkettige Triglyceride | 20 | 71±15 |
| 80 | Squalan | 20 | 57±12 |
| 80 | Cholesterin | 20 | 58±14 |
| 80 | Polyoxyethylenoleylether | 20 | 34±7 |
| 80 | Polysorbat 80 | 20 | 24±4 |
| 96 | Polysorbat 80 | 4 | 25±5 |

Als einziges Tensid führt Polysorbat 80 auch zu einer deutlichen Reduzierung der Teilchengröße, jedoch nur bei wirkstofffreien Liposomen (vgl. B. Kronberg et al., J. Pharm. Sci., 79 (8), 667-71 (1990). Bei wirkstoffhaltigen Liposomen, z.B. mit Tocopherolnicotinat, Hexachlorophen, führt dagegen ein Zusatz von Polysorbat (z.B. 20%) zu einer deutlichen Teilchengrößenerhöhung mit einer zusätzlich uneinheitlichen Verteilung, weshalb dieser Zusatz nicht geeignet ist zur Herstellung pharmazeutisch stabiler Produkte.

Wie erwähnt, erfolgt bei der erfindungsgemäßen Verwendung von fettsäureverestertem Collagenhydrolysat eine Reduzierung der Vesikelgröße auch bei gleichzeitiger Einarbeitung lipophiler Therapeutika. Als lipophile Wirkstoffe zur Einarbeitung können beispielsweise Aknetherapeutika wie Hexachlorophen, Tretinoin, oder Minocyclin verarbeitet werden. Aber auch der Einbau anderer topisch applizierbarer Wirkstoffe wie von alpha-Tocopherolnicotinat, Tromantadin-Base, Croconazol, Minocyclin, Meclocyclin, Cyproteron, Cyproteronacetat, Corticoiden, 2-tert.-Butyl-4-cyclohexylphenylnikotinat-N-oxid, Corticosteroiden, Androgenen, Ethinylöstradiol, nichtsteroidalen Antiphlogistika, Dihydropyridinen, Spironolacton, Erythromycinester, lipophilen Lokalanästhetika, Östradiolester oder lipophilen Antihistaminika ist möglich.

Der Wirkstoffgehalt kann entsprechend den therapeutischen Anforderungen variieren. So können z.B. pro 100 g mit fettsäureverestertem Collagenhydrolysat oder mit ÖKH versetztes Lipid 10 mg bis zu 50 g Wirkstoff eingesetzt werden. Damit ergibt sich die Möglichkeit, derartige Wirkstoffe in Liposomen mit sehr geringem Durchmesser zu verarbeiten. Aufgrund der allgemeinen Vesikelgröße ist eine Steigerung der therapeutischen Aktivität aufgrund des geringen Durchmessers möglich.

Besonders vorteilhaft bei dem Zusatz von fettsäureverestertem Collagenhydrolysat zu Lezithinliposomen ist, daß sich die gewünschte Teilchengröße durch unterschiedliche Zusatzmengen in einem weiten Bereich steuern läßt, wie dies aus der nachfolgenden Tabelle 2 ersichtlich ist.

**Tabelle 2**

| Einfluß des ÖKH-Anteils auf die Teilchengröße Tocopherolnikotinat-haltiger Liposomen (Herstellung: Hochdruckhomogenisator (30 min, 1,4·10⁸ Pa) | | |
|---|---|---|
| Konzentration Sojalezithin (mg/ml) | Konzentration ÖKH (mg/ml) | Teilchengröße (nm) |
| 100 | - | 51±14 |
| 80 | 20 | 42±17 |
| 50 | 50 | 25±5 |
| 20 | 80 | 26±5 |

Anhand nachfolgender Tabelle 3 kann man erkennen, daß der Zusatz von ÖKH auch bei zunächst größeren Liposomen, gewonnen durch die Herstellungsmethode Ethanolinjektion, eine Steuerung der Teilchengröße zuläßt.

**Tabelle 3**

| Einfluß des ÖKH-Anteils auf die Teilchengröße wirkstoff-freier Liposomen (Herstellung: Ethanolinjektion gemäß Beispiel 6) | | |
|---|---|---|
| Konzentration Sojalezithin (mg/ml | Konzentration ÖKH (mg/ml) | Teilchengröße (nm) |
| 32 | - | 637±309 |
| 32 | 8 | 175±106 |
| 24 | 12 | 109±26 |
| 24 | 24 | 99±26 |

Wie aus den Tabellen 2 und 3 ersichtlich ist, hängt bei wirkstoffhaltigen Liposomen das Ausmaß der Teilchengrößenreduktion vom Wirkstoff ab.

Wesentlich für eine breite Anwendung von Liposomen ist die bereits beschriebene Lagerungsstabilität der Liposomen. Wichtigster physikalischer Parameter für die Lagerungsstabilität ist die Teilchengröße der Vesikel. Die besonders bei Lezithinen auftretende Aggregation der Vesikel kann durch einen geeigneten Zusatz von fettsäureverestertem Collagenhydrolysat deutlich verringert werden, wie in nachfolgender Tabelle 4 gemäß Beispiel 2 nachgewiesen. Entsprechende Liposomen sind erfindungsgemäß daher sowohl primär kleiner, als auch bei Lagerung stabil. Ein evtl. aufgrund der Aggregation auftretender Wirkungsverlust kann so vermieden werden. Zusätzlich ist die Möglichkeit der Instabilität der Liposomen durch die Verringerung des Lezithinanteils (aufgrund der Zugabe von ÖKH) reduziert. Denn nur Lezithin selbst kann der destabilisierenden Lysolezithinbildung unterliegen. Die damit verbundene toxische Wirkung kann somit erheblich verringert werden.

In nachfolgender Tabelle 4 ist die Stabilität von erfindungsgemäßen Zubereitungen vergleichend getestet.

**Tabelle 4**

| Stabilität von ÖKH-haltigen Tocopherolnicotinatliposomen | | | | | |
|---|---|---|---|---|---|
| Lipid | Gesamtlipid konzentration (mg/ml) | Teilchengröße [nm] nach | | | |
| | | 0 | 4 | 12 | 24(Wochen) |
| Sojalezithin (Vgl.) | 100 | 90±19 | 96±23 | 196±188 | 274±191 |
| Sojalezithin/ÖKH 8:2 gemäß Beispiel 2(Erf.) | 100 | 49±14 | 45±14 | 44±10 | 42±11 |

Die erfindungsgemäßen Liposomen weisen also eine deutlich bessere Lagerungsstabilität auf als vergleichbare Liposome ohne ÖKH-Zusatz.

Die von B. Kronberg et al. a.a.O beschriebene Stabilisierung von Liposomen durch Polysorbat ist nur bei kurzer Lagerungszeit nachweisbar. Insbesondere wirkstoffhaltige Liposomen sind, wie bereits beschrieben, deutlich größer und inhomogener als entsprechende Liposomen aus Sojalezithin und fettsäureverestertem Collagenhydrolysat bzw. ÖKH. Bei vierwöchiger Lagerung der Liposomen kommt es zu einer deutlichen Sedimentbildung, die für ein pharmazeutisches Produkt nicht akzeptabel ist. Polysorbat ist daher als Zusatz für wirkstoffhaltige Liposomen mit dem Ziel der Teilchengrößenverringerung und der Vesikelstabilisierung nicht geeignet.

Für die topische Anwendung von Liposomenpräparationen werden der Liposomendispersion in der Regel polymere Gelbildner zur Viskositätserhöhung und damit der Verbesserung der Applikation eingesetzt. Hierzu werden insbesondere Polyacrylsäure (0,3-1,5%), Cellulosederivate (0,2-3%) und Natriumsalze von Acrylsäure-Acrylamidcopolymerisaten (z.B. im Handel erhältlich als Hostacerin PN73^{(R)}, 1-4%ige Verwendung) in der jeweils angegebenen Konzentration eingesetzt. Arbeitet man solche Gelbildner in Liposomendispersionen aus reinem Lezithin ein, so aggregieren die Vesikel häufig aufgrund von Wechselwirkungen mit dem Polymer. Die erfindungsgemäßen fettsäureveresterten Collagenhydrolysat-haltigen Vesikel zeigen dagegen überraschenderweise nur geringfügige Teilchengrößenänderungen nach Verarbeitung zu Polymergelen, wie in nachfolgender Tabelle 5 dokumentiert.

**Tabelle 5**

| Teilchengrößenänderung nach Einarbeitung von Liposomen in Hostacerin^{(R)}-Gele und anschließender Lagerung bei 21°C | | | | | | | |
|---|---|---|---|---|---|---|---|
| Lipid | Gesamtlipidkonz. (mg/ml) | Teilchengröße vor Einarb. (nm) | Teilchengröße nach Einarbeitung (nm) | Lagerungsdauer (Wochen) | | | |
| | | | | 5 | 12 | 24 | 32 |
| SL | 100 | 95±19 | 108±23 | 110±24 | 117±23 | 124±23 | 142±31 |
| SL/ÖKH | 100 | 40±10 | 40±10 | 42±10 | 45±10 | 42±11 | 41±9 |
| SL = Sojalezithin | | | | | | | |

Die erfindungsgemäß hergestellte Dispersion kann anschliessend mit üblichen Mengen eines geeigneten Gelbildners, vorzugsweise mit einem der oben beschriebenen, vermischt werden. Das Gel wird dabei in üblicher bekannter Weise bereitet.

Besonders bevorzugt zur Herstellung der erfindungsgemäßen Zubereitungen sind Mischungen aus Ei- oder Sojalezithin mit Collagenhydrolysat, insbesondere ÖKH, sowie den genannten Gelbildnern und Wirkstoffen.

Wahlweise können übliche Zusatzstoffe, wie beispielsweise ein Antioxidans, z.B. Vitamin E oder Butylhydroxytoluol, und Konservierungsmittel wie Phenoxethol, Sorbinsäure, Kathon CG oder Parabene in üblichen Mengen zugesetzt werden.

Die auf diese Weise hergestellten wirkstoffhaltigen Liposomenzubereitungen weisen eine Vesikelgröße von etwa 20 bis 150 nm, vorzugsweise 20 bis 70 nm, auf. Sie eignen sich inbesondere zur topischen Anwendung - je nach Wirkstoff - als Dermatologika oder Kosmetika. Dabei ist gegenüber bisher bekannten Produkten eine Steigerung der therapeutischen Aktivität zu verzeichnen, da aufgrund des geringeren Vesikeldurchmessers eine vermehrte Interaktion mit den Zellen erfolgt und ferner auch die Stabilität der Zubereitung erhöht ist, so daß eine langanhaltende gleichbleibende Wirksamkeit ohne toxische Nebenwirkungen gewährleistet ist.

Ferner können die erfindungsgemäßen Zubereitungen einfach hergestellt werden, wobei die genannten Vorteile bei Verzicht auf hydrierte Lipide genutzt werden können.

Die nachfolgenden Beispiele erläutern die vorliegende Erfindung.

### Beispiel 1

### Herstellung eines ÖKH-haltigen Liposomengels mittels Hochdruckhomogenisator

In ein geignetes Gefäß werden 80 g Sojalezithin, 9 g Phenoxyethanol und 45 g Ethanol unter Rühren auf 50°C erwärmt. In die klare homogene Flüssigkeit werden 400 mg Butylhydroxytoluol, 20 g ÖKH und 745,6 g hypotoner Puffer, pH 6,5, gegeben. Die entstandene Lipiddispersion (900 g) wird mit einem Hochdruckhomogenisator behandelt (1,1·10⁸ Pa, 40 min) und anschließend durch eine Membran (0,45 »m) filtriert.

Zur Herstellung eines Gels werden 5 g Polyacrylsäure (z.B. Carbopol^{(R)} 984), 1 g Phenoxyethanol und 8,8 g TRIS in einer Mischung aus 5 g Ethanol und 80,2 g Phosphatpuffer pH 6,5 dispergiert. Nach einem Tag Ausquellung wird das Gelkonzentrat mit der oben beschriebenen Liposomendispersion vermischt.

### Beispiel 2

### Herstellung eines ÖKH-haltigen Tocopherolnicotinat-Liposomengels mittels Hochdruckhomogenisator

In ein geeignetes Gefäß werden 80 g Sojalezithin, 20 g α-Tocopherolnicotinat, 7,5 g Phenoxyethanol und 37,5 g Ethanol unter Rühren auf 50°C erwärmt. In die klare homogene Flüssigkeit werden 400 mg Butylhydroxytoluol, 20 g ÖKH und 584,6 g hypotoner Phosphat-Puffer pH 6,5 gegeben. Die entstandene Lipiddispersion (750 g) wird mit einem Hochdruckhomogenisator behandelt (1,1·10⁸ Pa, 40 min) und anschließend durch eine Membran (0,45 »m) filtriert (vgl. Tabelle 4).

Zur Herstellung eines Gels werden 25 g Hostacerin^{(R)} und 2,5 g Phenoxyethanol in einer Mischung aus 12,5 g Ethanol und 210 g Phosphatpuffer pH 6,5 dispergiert. Nach einem Tag Ausquellung wird dieses Gelkonzentrat mit der oben beschriebenen Liposomendispersion vermischt. Diese Liposomen weisen nach Lagerung bei 21°C eine deutlich bessere Stabilität auf als vergleichbare Liposomen ohne ÖKH-Zusatz (vgl. Tabelle 5).

### Beispiel 3

### Herstellung eines ÖKH-haltigen Cyproteron-Liposomengels mittels Hochdruckhomogenisator

In ein geeignetes Gefäß werden 80 g Sojalezithin, 100 mg Cyproteron, 10 g Phenoxyethanol und 50 g Ethanol unter Rühren auf 50°C erwärmt. In die klare homogene Flüssigkeit werden 400 mg Butylhydroxytoluol, 20 g ÖKH und 829,5 g hypotoner Phosphat-Puffer pH 6,5 gegeben. Die entstandene Lipiddispersion (990 g) wird mit einem Hochdruckhomogenisator behandelt (1,1·10⁸ Pa, 40 min) und anschließend durch eine Membran (0,45 »m) filtriert. Die hergestellten Liposomen weisen eine deutlich bessere Lagerungsstabilität auf als vergleichbare Liposomen ohne ÖKH-Zusatz.

Zur Herstellung eines Gels werden 10 g Carboxymethylcellulose unter Rühren auf 990 g Liposomendispersion aufgestreut und 24 Stunden quellen gelassen.

### Beispiel 4

### Herstellung eines ÖKH-haltigen Tretinoin-Liposomengels mittels Hochdruckhomogenisator

In einem geeigneten Gefäß werden 200 mg Tretinoin und 400 mg Butylhydroxytoluol in 50 g Ethanol unter Rühren gelöst. In isotonen Phosphatpuffer pH 6,5 (802 g) werden 80 g Lezithin, 20 g ÖKH und 10 g Phenoxyethanol dispergiert. Die ethanolische Lösung wird unter Ultraturraxbehandlung in die wäßrige Phase eingerührt. Die entstandene Dispersion (962,5 g) wird mit einem Hochdruckhomogenisator behandelt (1,1·10⁸ Pa, 40 min) und anschließend durch eine Membran (0,45 »m) filtriert. Die hergestellten Liposomen weisen eine Teilchengröße von 23±4 nm auf.

Zur Herstellung eines Gels werden 12,5 g Polyacrylsäure (Carbomer 941^{(R)}) und 25 g TRIS unter Rühren in der Liposomendispersion dispergiert und 24 Stunden quellen gelassen.

### Beispiel 5

### Herstellung eines ÖKH-haltigen Croconazol-Liposomengels mittels Hochdruckhomogenisator

In einem geeigneten Gefäß werden 1 g Croconazol in 10 g Ethanol unter Rühren gelöst. Die ethanolische Lösung wird unter Ultraturraxbehandlung in die wäßrige Phase, enthaltend Lezithin, ÖKH, Phenoxethol, eingerührt. Die entstandene Dispersion wird mit einem Hochdruckhomogenisator behandelt (1,1·10⁸ Pa, 40 min) und anschließend durch eine Membran (0,45 »m) filtriert.

Zur Herstellung eines Gels werden 12,5 g Polyacrylsäure (Carbomer 941^{(R)}) und 25 g TRIS unter Rühren in der Liposomendispersion dispergiert und 24 Stunden quellen gelassen. Der pH-Wert des Gels liegt zwischen 7 und 8. Die Teilchengröße der darin enthaltenen Liposomen beträgt 22±3 nm.

### Beispiel 6

### Herstellung einer ÖKH-haltigen Liposomendispersion mittels Ethanolinjektion

2,4 g Sojalezithin und 2,4 g ÖKH werden in 20 ml Ethanol gelöst und mittels einer geeigneten Spritze in 80 ml isotonen Puffer pH 6,5 injiziert. Die Injektion erfolgt in einem Ultraschallbad zur besseren Verteilung der ethanolischen Lösung. Zur Reinigung wird die Dispersion durch eine Membran (0,45 »m) filtriert. Die Endkonzentration beträgt 4,8 % Lipid und 20 % Ethanol. Auch bei dieser Herstellungstechnik werden im Vergleich zu reinen Sojalezithinliposomen kleinere Vesikel erhalten (vgl. Tabelle 3).

### Beispiel 7

### Herstellung Hexachlorophenhaltiger Liposomen mittels Ethanolinjektion

1,0 g Hexachlorophen, 0,2 g Tocopherol, 2,5 g Sojalezithin und 2,5 g ÖKH werden in 20 g Ethanol gelöst und mittels einer geeigneten Spritze in 74 ml Citrat/Phosphat-Puffer pH 5,5 (mit 0,2 % Sorbinsäure/Kaliumsorbat) unter Ultraschallbehandlung injiziert. Zur Reinigung wird die Dispersion durch eine Membran (0,45 »m) filtriert. Die Endkonzentration beträgt 1% Hexachlorophen, 5,0% Lipid und 20% Ethanol, die Teilchengröße 70-135 nm. Aus der Liposomendispersion wird mit 1 g Polacrylsäure (Carbopol 984^{(R)}) ein Gel bereitet. Die Teilchengröße der Liposomen in der Gelzubereitung beträgt 100-110 nm.

## Patentansprüche

1. Verfahren zur Herstellung einer Liposomendispersion enthaltend 0,5 bis 35 Gew.-%, bezogen auf die Dispersion, an Lezithin und fettsäureverestertem Collagenhydrolysat, übliche Zusatzstoffe sowie Wasser, wobei die Vesikel aus einer Mischung aus einem oder mehreren Lezithinen mit fettsäureverestertem Collagenhydrolysat aufgebaut sind und eine Teilchengröße von 20 bis 150 nm aufweisen, dadurch gekennzeichnet, daß
a) das Lezithin mit mehr als 15 bis 90%, vorzugsweise 20 bis 80%, fettsäureverestertem Collagenhydrolysat (bezogen auf die Gesamtmenge an Lezithin und fettsäureverestertem Collagenhydrolysat), und gegebenenfalls mit einem pharmazeutischen Wirkstoff sowie üblichen Zusatzstoffen in einer wäßrigen Phase bei einem pH-Wert von 5 bis 7 in Gegenwart eines für diesen pH-Wert-Bereich wirksamen Puffers dispergiert wird und die erhaltene Dispersion in einem Hochdruckhomogenisator zerkleinert und homogenisiert wird oder
b) zur Dispergierung eine Mischung aus dem Lezithin, dem fettsäureveresterten Collagenhydrolysat in den angegebenen Mengen und gegebenenfalls pharmazeutischem Wirkstoff und üblichen Zusatzstoffen sowie Ethanol in eine wäßrige Phase bei pH 5-7 in Gegenwart eines für diesen pH-Wert-Bereich wirksamen Puffers injiziert wird.

2. Verfahren gemäß Anspruch 1, dadurch gekennzeichnet, daß man als Lezithin Soja- oder Eilezithin verwendet.

3. Verfahren gemäß Ansprüch 1 oder 2, dadurch gekennzeichnet, daß man als fettsäureverestertes Collagenhydrolysat ölsäureverestertes Collagenhydrolysat verwendet.

4. Verfahren gemäß einem der Ansprüche 1 bis 3, dadurch gekennzeichnet, daß als Wirkstoff Hexachlorophen, Tretinoin, Minocyclin, Meclocyclin, α-Tocopherolnicotinat, Tromantadin-Base, Croconazol, Cyproteron, Cyproteronacetat, Corticoide, 2-tert.-Butyl-4-cyclohexylphenylnikotinat-N-oxid, Corticosteroide, Androgene, Ethinylöstradiol, nichtsteroidale Antiphlogistika, Dihydropyridine, Spironolacton, Erythromycinester, lipophile Lokalanästhetika, Östradiolester oder lipophile Antihistaminika verwendet werden.

5. Verfahren gemäß einem der Ansprüche 1 bis 4, dadurch gekennzeichnet, daß die Vesikel eine Größe von 20 bis 70 nm aufweisen.

6. Verfahren zur Herstellung einer Liposomenzubereitung, enthaltend eine Dispersion, erhalten nach dem Verfahren gemäß einem der Ansprüche 1 bis 5, dadurch gekennzeichnet, daß der Dispersion ein Gelbildner, ausgewählt aus Gelbildnern auf Basis Polyacrylsäure, eines Natriumsalzes eines Acrylsäure-Acrylamidcopolymerisats oder eines Cellulosederivats zugesetzt wird.

7. Verwendung einer Liposomendispersion, hergestellt nach dem Verfahren gemäß einem der Ansprüche 1 bis 5, zur Herstellung eines topischen Mittels zur kosmetischen oder dermatologischen Anwendung beim Menschen.

## Claims

1. A process for the manufacture of a liposome dispersion containing 0.5 to 35% by weight, based on the dispersion, of lecithin and collagen hydrolyzate esterified with fatty acid, conventional additives and water, the vesicles being made up of a mixture of one or more lecithins with collagen hydrolyzate esterified with fatty acid, and having a particle size of 20 to 150 nm, characterized in that
a) lecithin is dispersed with more than 15 to 90%, preferably 20 to 80%, of collagen hydrolyzate esterified with fatty acid (based on the total amount of lecithin and collagen hydrolyzate esterified with fatty acid), and optionally with a pharmaceutical active ingredient and conventional additives, in an aqueous phase at a pH of 5 to 7 in the presence of a buffer effective for this pH range, and the resulting dispersion is comminuted and homogenized in a high-pressure homogenizer, or
b) to produce a dispersion, a mixture of lecithin, collagen hydrolyzate esterified with fatty acid, in the indicated amounts, and optionally a pharmaceutical active ingredient, conventional additives and ethanol, is injected into an aqueous phase at pH 5-7 in the presence of a buffer effective for this pH range.

2. A process according to Claim 1, characterized in that soya or egg lecithin is used as the lecithin.

3. A process according to Claim 1 or 2, characterized in that collagen hydrolyzate esterified with oleic acid is used as the collagen hydrolyzate esterified with fatty acid.

4. A process according to one of Claims 1 to 3, characterized in that hexachlorophene, tretinoin, minocycline, meclocycline, α-tocopherol nicotinate, tromantadine base, croconazole, cyproterone, cyproterone acetate, corticoids, 2-tert-butyl-4-cyclohexylphenyl nicotinate N-oxide, corticosteroids, androgens, ethynyloestradiol, non-steroidal antiphlogistics, dihydropyridines, spironolactone, erythromycin esters, lipophilic local anaesthetics, oestradiol esters or lipophilic antihistamines are used as the active ingredient.

5. A process according to one of Claims 1 to 4, characterized in that the vesicles have a size of 20 to 70 nm.

6. A process for the manufacture of a liposome preparation containing a dispersion obtained by the process according to one of Claims 1 to 5, characterized in that a gelling agent, selected from gelling agents based on polyacrylic acid, on a sodium salt of an acrylic acid/acrylamide copolymer or on a cellulose derivative, is added to the dispersion.

7. Use of a liposome dispersion manufactured by the process according to one of Claims 1 to 5 for the manufacture of a topical composition for cosmetic or dermatological application to humans.

## Revendications

1. Procédé de préparation d'une dispersion de liposomes contenant de 0,5 à 35 % en poids, par rapport à la dispersion, de lécithine et d'hydrolysat de collagène estérifié à l'acide gras, des additifs habituels ainsi que de l'eau, les vésicules étant composées d'un mélange d'une ou plusieurs lécithines et de l'hydrolysat de collagène estérifié à l'acide gras et présentant une taille des particules comprise entre 20 et 150 nm, caractérisé en ce que
a) la lécithine est dispersée avec plus de 15 jusqu'à 90 %, de préférence 20 à 80 % d'hydrolysat de collagène estérifié à l'acide gras (par rapport à la quantité totale de lécithine et d'hydrolysat de collagène estérifié à l'acide gras), et, le cas échéant, avec une substance active pharmaceutique et des additifs habituels, dans une phase aqueuse d'une valeur de pH comprise entre 5 et 7 en présence d'une solution tampon efficace pour lesdites valeurs de pH, et que la dispersion obtenue est fragmentée et homogénéisée dans un homogénéisateur à haute pression, ou
b) pour la dispersion, un mélange de la lécithine, de l'hydrolysat de collagène estérifié à l'acide gras dans les quantités indiquées et, le cas échéant, de la substance active pharmaceutique et d'additifs habituels ainsi que d'éthanol est injecté dans une phase aqueuse de pH 5 à 7 en présence d'une solution tampon efficace pour lesdites valeurs de pH.

2. Procédé selon la revendication 1, caractérisé en ce qu'on utilise comme lécithine de la lécithine de soja ou d'oeuf.

3. Procédé selon la revendication 1 ou 2, caractérisé en ce qu'on utilise comme hydrolysat de collagène estérifié à l'acide gras de l'hydrolysat de collagène estérifié à l'acide oléique.

4. Procédé selon l'une quelconque des revendications 1 à 3, caractérisé en ce que la substance active est choisie parmi les substances suivantes: hexachlorophène, trétinoïne, minocycline, méclocycline, α-tocopherolnicotinate, base de tromantadine, croconazole, cyprotérone, cyprotéroneacétate, corticoïdes, 2-tert-butyl-4-cyclohexylphénylnicotinate-N-oxyde, corticostéroïdes, androgènes, éthinyl-oestradiol, antiphlogistiques non-stéroïdiens, dihydropyridines, spironolactone, erythromicyneester, anesthésiques locaux lipophiles, oestradiolester ou antihistaminiques lipophiles.

5. Procédé selon l'une quelconque des revendications 1 à 4, caractérisé en ce que les vésicules présentent une taille comprise entre 20 et 70 nm.

6. Procédé de fabrication d'une préparation de liposomes contenant une dispersion obtenue selon le procédé conforme à l'une des revendications 1 à 5, caractérisé en ce qu'on ajoute à la dispersion un gélificateur choisi parmi les gélificateurs à base d'acide polyacrylique, d'un sel de sodium d'un copolymère d'acide acrylique/amide acrylique ou d'un dérivé de cellulose.

7. Utilisation d'une préparation de liposomes fabriquée selon le procédé conforme à l'une des revendications 1 à 5, pour fabriquer un moyen topique destiné à l'application cosmétique ou dermatologique chez l'homme.
